(19) Europäisches Patentamt
European Patent Office
Office européen des brevets

(11) Publication number: **0 447 362 A1**

(12) # EUROPEAN PATENT APPLICATION

(21) Application number: **91810154.4**

(22) Date of filing: **07.03.91**

(51) Int. Cl.[5]: **A61K 31/785**, A61K 9/20, A61K 9/16, A61K 47/12

A request for correction, renumbering of the claims ( 1 to 11 instead of 1 to 10 ), has been filed pursuant to Rule 88 EPC. A decision on the request will be taken during the proceedings before the Examining Division (Guidelines for Examination in the EPO, A-V, 2.2).

(30) Priority: **13.03.90 US 493039**

(43) Date of publication of application:
**18.09.91 Bulletin 91/38**

(84) Designated Contracting States:
**DE DK ES FR GB GR IT**

(71) Applicant: **WARNER-LAMBERT COMPANY**
**201 Tabor Road**
**Morris Plains New Jersey 07950 (US)**

(72) Inventor: **Hussein, Mamoun M.**
**115 Boulevard**
**Mountain Lakes, N.J. 07046 (US)**
Inventor: **Killeen, Michael J.**
**402 Newton-Sparta Road, Rte 1, Boc 269**
**Lafayette N.J. 07848 (US)**
Inventor: **Sheu, Shan Shan**
**44 S-14 Center Grove Road**
**Randolph, N.J. 07869 (US)**
Inventor: **Yang, Robert K.**
**12 Roc Etam Road**
**Randolph, N.J. 07869 (US)**

(74) Representative: **Silbiger, Jakob, Dr.**
**c/o CAPSUGEL AG Fabrikmattenweg 2-4**
**CH-4144 Arlesheim (CH)**

(54) **Improved ingestible anion exchange resin delivery system compositions containing adipic acid.**

(57) Improved ingestible anion exchange resin delivery system compositions comprising anion exchange resin and a confectionery material, wherein the improvement comprises from about 0.1% to about 5.0% by weight based on total weight of composition of adipic acid which is homogenously incorporated into said composition.

EP 0 447 362 A1

# IMPROVED INGESTIBLE ANION EXCHANGE RESIN DELIVERY SYSTEM COMPOSITIONS CONTAINING ADIPIC ACID

Ingestible delivery systems for actives are known in the art and are useful for the delivery of actives such as fiber and drugs. These delivery systems can be beneficial in masking the unpleasant taste and mouthfeel of the fiber and/or drug and this benefit encourages patient compliance with a recommended therapy.

U. S. Patent No. 3,974,272 describes oral palatable drug formulations containing aqueous media and cholestyramine. Also described are a method of treating hyper-cholestrolemia and chewable products containing cellulosic/gum colloids.

U.S. Patent No. 4,778,676 describes a chewable delivery system composition for actives comprising (a) a pre-coated active and (b) a confectionery matrix comprising a binder system comprising gelatin, a humectant material, a sweetener and water. The active can be cholestyramine and the composition typically contains 0.3-1.5% by weight citric acid.

U.S. Patent No. 4,818, 539 describes an ingestible aggregate comprising a pre-swelled, anhydrous hydrocolloid and a substrate. The substrate can be a dietary fiber or a drug such as cholestyramine. Hydrocolloids include natural and modified gums, celluloses, pectins, mucillages, modified starches and mixtures thereof. These compositions may also contain food grade acids (such as citric acid), flavors, sweeteners, colors and other conventional additives.

A disadvantage with the prior art anion exchange resin delivery systems is stickiness and tackiness of the material, particularly during processing when citric acid is included in the formulation. This tackiness can lead to gumming and foiling of the processing equipment and excessive softness and stickiness of the product. The present invention eliminates the tackiness and stickiness problems associated with confectionery-based anion exchange resin delivery systems by incorporating adipic acid therein. Thus, it is an object of this invention to provide improved ingestible anion exchange resin delivery compositions which are not tacky and sticky during processing or in the final product. It is a further object of this invention to provide an improved process for making non-tacky anion exchange resin compositions. It is an even further object of this invention to provide non-tacky anion exchange resin delivery system compositions which have a pleasant taste, acceptable mouthfeel, and reduced acidity.

This invention relates to improved ingestible anion exchange resin delivery system compositions and the process for preparing same. The present invention provides an ingestible anion exchange resin delivery system composition comprising anion exchange resin and a confectionery material, characterised in that adipic acid is homogeneously incorporated into the composition in an amount of from about 0.1% to about 5.0% by weight of the total composition. The confectionery material preferably comprises a gelatin-frappe which may optionally comprise sweeteners, flavorings colorings, humectants, fillers, emulsifiers, thickeners and buffers. The anion exchange resin preferably is selected from, cholestyramine, colestipol and anion exchange resins having an imidazolium group and is preferably coated with lecithin. Incorporation of adipic acid into these compositions result in improvements in taste and reductions in product acidity and tackiness.

The present invention involves improvements in ingestible anion exchange resin delivery system compositions. In these ingestible delivery system compositions comprising an anion exchange resin and a confectionery material, the improvement of this invention comprises about 0.1% to about 5.0% by weight of adipic acid based on total weight of the composition, said adipic acid being homogenously incorporated into said composition. The invention results in anion exchange resin delivery system compositions which have improved taste and reduced tackiness and acidity.

The preparation of anion exchange resin compositions useful according to this invention is described in U.S. Patent No. 4,778,676, which disclosure is hereby incorporated by reference and made a part of this application. The anion exchange resins useful in this invention are the edible anion exchange resins having anticholesteremic properties that combine with bile acids in the intestines and are excreted and eliminated from the body. Preferred anion exchange resins include cholestyramine, colestipol and anion exchange resins having the imidazolium group as the functional group. Preferred resins of the imidazolium type are described in U.S. Patent No. 4,557,930, which disclosure is hereby incorporated by reference and made a part of this application. The most preferred anion exchange resin for use in this invention is cholestyramine.

The confectionery material can be any of the various edible matrices known in the art such as, for example, the gel-type, candy bar type, boiled candy, chewable mint or tablet, nougat or other candy or confectionery forms. The preferred confectionery material is a gel-type confectionery material which is generally comprised of a gel-forming material, water, sweeteners, flavorings, buffers and acidulents. Useful gel-forming materials include gelatin, glycerin, pectin, gum arabic gels, starches, carrageenan gels, locust bean gum, xanathan gum and the like and mixtures thereof. Preferably the confectionery material comprises gelatin or a mixture of gelatin,

water and glycerin. Most preferably the confectionery material is a textured gelatin frappe.

Because of its unpleasant taste and mouthfeel, it is preferred that the anion exchange resin be pre-coated to mask or disguise these unpleasant characteristics. The anion exchange resin is preferably coated with lecithin, polyoxyalkylenes, polyalkylene glycols, glycerides, waxes or mixtures thereof. Most preferably the anion exchange resin is coated with lecithin.

The compositions of this invention contain adipic acid preferably in an amount of from about 0.1% to about 5.0% by weight based on total weight of the composition; more preferably from about 0.2% to about 1.0%. Adipic acid (also known as hexanedioic acid and 1,4-butanedicarboxylic acid) has the following chemical structure:

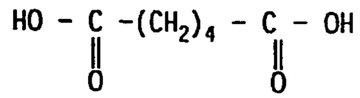

It is produced commercially by the nitric acid oxidation of cyclohexanol or a mixture of cyclohexanol and cyclohexane.

Adipic acid is less acidic and less hygroscopic than the commonly-used acidulent (e.g. citric acid) in confectionery delivery compositions. The use of adipic acid in the compositions of this invention is believed responsible for dramatically reducing the tackiness and stickiness of the compositions and for imparting a pleasant taste.

Adipic acid may be incorporated into the compositions in solid or liquid form. Preferably the adipic acid is encapsulated, such as for example, with maltodextrin or hydrogenated vegetable oil or the like.

The composition of this invention preferably comprises from about 1% to about 40% by weight of the anion exchange resin, more preferably from about 10% to about 30%. The composition may additionally contain one or more optional ingredients such as sweeteners, flavorings, colorings, humectants, fillers, emulsifiers, thickeners, buffers and other conventional additives well known in the confectionery art. Additional ingredients may also include medicaments, vitamins, mineral supplements and other chemical or biological substances, such as laxatives, antacids, analgesics, appetite suppressants, anti-inflammatory substances, coronary dilators, cerebral dilators, peripheral vasodilators, anti-infectives, psychotropics, antimanics, stimulants, decongestants, gastro-intestinal sedatives, antidiarrheal preparations, anti-anginal drugs, vasodilators, anti-arrythmics, anti-hypertensive drugs, vasoconstrictors and migrane treatments, antibiotics, tranquilizers, antipsychotics, antitumor drugs, anticoagulants and antithrombotic drugs, hypnotics, sedatives, anti-emetics, anti-nauseants, anti-convulsants, neuromuscular drugs, hyper- and hypoglycaemic agents, thyroid and antithyroid preparations, diuretics, antispasmodics, uterine relaxants, nutritional additives, antiobesity drugs, anabolic drugs, erythropoietic drugs, antiasthmatics, expectorants, cough suppressants, mucolytics, anti-uricemic drugs, and the like. Mixtures of the drugs and medicaments may also be used.

Flavors which may optionally be added to the delivery system are those well known in the confectionery art. For example, synthetic flavor oils, and/or oils derived from plants, leaves, flowers, fruits and so forth, and combinations thereof, are useful. The flavors may be used in liquid, solid and/or encapsulated forms.

Representative flavor oils include spearmint oil, peppermint oil, cinnamon oil, and oil of wintergreen (methyl salicylate). Also useful are artificial, natural or synthetic fruit flavors such as citrus oils including lemon, orange, grape, lime, and grapefruit, and fruit essences including apples, strawberry, cherry, pineapple and so forth.

The amount of flavoring agent employed is normally a matter of preference subject to such factors as flavor type, base type and strength desired. In general, amounts of about 0.05% to about 5.0% by weight of the final product are useful with amounts of about 0.3% to about 1.5% being preferred and about 0.8% to about 1.2% being most preferred. Sweetening agents may be selected from a wide range of materials such as water-soluble sweetening agents, water-soluble artificial sweeteners, and dipeptide based sweeteners, including mixtures thereof. Without being limited to particular sweeteners, representative illustrations encompass:

A. Water-soluble sweetening agents such as monosaccharides, disaccharides and polysaccharides such as xylose, ribose, glucose, mannose, galactose, fructose, dextrose, sucrose, sugar, maltose, partially hydrolyzed starch, or corn syrup solids and sugar alcohols such as sorbitol, xylitol, mannitol and mixtures thereof.

B. Water-soluble artificial sweeteners such as the soluble saccharin salts, i.e., sodium or calcium saccharin salts, cyclamate salts, acesulfame-K and the like, and the free acid form of saccharin.

C. Dipeptide based sweeteners such as L-aspartyl L-phenylalanine methyl ester and materials described in U.S. Patent No. 3,492,131 and the like. In general, the amount of sweetener will vary with the desired sweetener selected for a particular composition. The amount will normally be about 0.01% to about 90% by weight when using an easily extractable sweetener. The water-soluble sweeteners described in category

A are preferably used in amount of about 25% to about 75% by weight, and most preferably about 50% to about 65% by weight of the final composition. In contrast, the artificial sweeteners described in categories B and C are used in amounts of about 0.005% to about 5.0% and most preferably about 0.05% to about 2.5% by weight of the final delivery system. These amounts are necessary to achieve a desired level of sweetness independent from the flavor level achieved from the flavor oil.

A preferred process for preparing the anion exchange resin delivery compositions of this invention comprises initially preparing a pre-blend of the anion exchange resin and the coating material, such as lecithin. In carrying out this process the anion exchange resin is coated with the coating material at a weight ratio of coating to resin, from about 11 to about 99:1. For example, cholestyramine resin granules are placed in a mixer and lecithin is then added and the ingredienta are agitated until the resin granules are completely coated with lecithin. The confectionery material (preferably a gelatin-frappe) can be prepared by adding gelatin and glycerin to deionized water and heating the ingredients to dissolve the gelatin and glycerin. Fructose, sorbitol, and adipic acid can be added to the gelatin/glycerin solution and the material mixed until a fluffy, textured frappe results. Preferably the gelatin-frappe has a pH of about. 3.6 or higher.

The pre-blend consisting of the coated anion exchange resin is then added to the gelatin-frappe along with pectin and selected flavorings. This mixture is then uniformly mixed preferably at high speed for about 1-10 minutes. The product is then removed from the mixer and stored for approximately 12 hours or more to allow the product to set-up. The product is subsequently apportioned into the desired shape and size, such as bar-shaped portions, and packaged.

Other processes which are conventional and known in the confectionery art can be used to prepare the compositions of this invention. The equipment and apparatus useful in accordance with the present invention are well known in the confectionery manufacturing art and the selection of a specific apparatus will be apparent to the skilled artisan.

The following examples are presented to illustrate this invention and are not intended in a limitative sense. All parts and percentages used in the examples are on a weight basis unless otherwise indicated.

Example I

A composition within the scope of this invention was prepared as follows: 24.5 kilograms (kg) of cholestyramine resin were added to a mixer and mixed at low speed. 4.9 kg of lecithin were quickly added to the mixer and the ingredients were mixed at high speed for approximately 10 minutes at about 54°C to make a Resin Pre-Blend.

3.6 kg of deionized water were added to a stainless steel container and heated to 80°C. Then 300 grams (g) of gelatin and 1.5 kg of glycerine were added to the container and mixed while maintaining a temperature of about 75°C to form a gelatin/glycerine solution. To a mixer are added 13.22 kg of fructose crystals and 3.6 kg of sorbitol crystals, and these ingredients were mixed for about 2 minutes at low speed and a temperature of about 30°C. Then the gelatin/glycerine solution prepared above was rapidly added to the mixer and mixed for approximately 5 minutes at a temperature of about 34°C to form a gelatin frappe having a pH of about 3.9. Next, 7.2 kg of the Resin Pre-Blend prepared above were added to this gelatin-frappe, along with 432.0 grams of pectin, 60.0 grams of adipic acid and 120.0 grams of flavoring. These ingredients were mixed for approximately 3.5 minutes at a temperature of about 40°C. The final bulk product was stored in a plastic-lined container for at least 24 hours and then extruded into bars of approximately 20 grams in weight. The bulk product and the extruded bars were evaluated for tackiness by visual and physical examination. The results are presented below:

| | Tackiness Rating |
|---|---|
| Bulk Product | 2 |
| Extruded Bar | 2 |

(Scale 1-5)
(1=least tackiness;
5=most tackiness)

Example II

Following the procedures of Ex. 1, a composition within the scope of this invention was prepared, except that 99.0 grams of 70% adipic acid encapsulated in 30% maltodextrin were used instead of the 60.0 grams of adipic acid. The gelatin frappe had a pH of about 3.9-4.47. The final bulk product and extruded bars were evaluated for tackiness as in Ex. 1 and were comparable to the product of Ex. 1.
No tackiness or stickiness was noted in the product.

Example III (Comparative)

A composition falling outside this invention and containing no adipic acid was prepared as follows:
A Resin Pre-Blend was prepared by adding 15.0 kg of cholestyramine resin and 30 kg of lecithin to a mixer and mixing at high speed at a temperature of about 40-50°C.

3.0 kg of deionized water, 1.25 kg of glycerine and 250 grams of gelatin were added to a mixer and mixed at high speed for approximately one minute at a temperature of about 54°C. Next 10.94 kg of fructose crystals and 3.0 kg of sorbitol crystals were added to this mixer and mixed for about two minutes at a temperature of about 28°C to form a gelatin frappe having a pH of about 4.1. Then 6.0 kg of the Resin Pre-Blend prepared above were added to this mixer along with 360.0 grams of pectin and 240.0 grams of flavoring. The ingredients were then mixed at high speed for approximately two minutes at a temperature of about 32°C. The final bulk product was stored in a plastic-lined container for at least 24 hours and extruded in bars having approximately 22 grams in weight.

The bulk product was evaluated for tackiness as in Ex. 1 and the results are presented below:

| | Tackiness Rating (Average) |
|---|---|
| Bulk Product | 2 |

(Scale: 1=least tackiness, 5=most tackiness)

Example IV (Comparative)

A composition falling outside the scope of this invention and containing citric acid was prepared as follows:
A Resin Pre-Blend was prepared by adding 24.5 kg of cholestyramine resin and 4.9 kg of lecithin to a mixer and mixing at high speed for approximately 10 minutes at a temperature of about 50°C.

3.6 kg of deionized water were added to a stainless steel container and heated to about 80°C. Next, 300 grams of gelatin and 1.5 kg of glycerine were added and mixed while maintaining a temperature of about 75°C. Then 13.19 kg of fructose crystals, 3.6 kg of sorbital crystal and 60 grams of citric acid were added to a mixer and mixed for about 20 minutes at a temperature of about 34°C. The gelatin/glycerine solution prepared above was rapidly added to this mixer and the ingredients mixed for approximately five minutes at a temperature of about 28°C to form a gelatin frappe. The gelatin frappe had a pH of about 2.8.

Next, 7.2 kg of the Resin Pre-Blend prepared above were added to the gelatin-frappe, along with 432.0 grams of pectin and 120.0 grams of flavoring. These ingredients were mixed for approximately 3 1/2 minutes at a temperature of about 33°C. The final bulk product was stored in a plastic-lined container for at least 24 hours and then extruded in bars of approximately 20 grams in weight. The bulk product was evaluated for tackiness as in Ex. 1. The results are presented below:

| | Tackiness Rating (Average) |
|---|---|
| Bulk Product | 4.5 |

Scale:
(1=least tackiness
5=most tackiness)

In summary, the invention provides:

1. In an ingestible anion exchange resin delivery system composition comprising anion exchange resin and

a confectionery material, the improvement comprising based on total weight of said composition about 0.1% to about 5.0% by weight of adipic acid, said adipic acid being homogenously incorporated into said composition.

2. The composition of 1 comprising from about 0.2% to about 1% by weight adipic acid.

3. The composition of 1 wherein said anion exchange resin is selected from the group consisting of cholestyramine, colestipol and anion exchange resins having an imidazolium group.

4. The composition of 1 wherein said anion exchange resin is coated with lecithin, polyoxyalkylenes, glycerides, polyalkyleneglycols, waxes or mixtures thereof.

5. The composition of 1 wherein said confectionery material comprises gelatin.

6. The composition of 1 wherein said confectionery material is a mixture of gelatin, water and glycerin.

7. The composition of 1 wherein said confectionery material is a gelatin-frappe.

8. The composition of 1 additionally comprising one or more material(s) selected from the group consisting of sweeteners, flavoring~, colorings, humectants, fillers, emulsifiers, thickeners and buffers.

9. The composition of 1 wherein said anion exchange resin comprises about 1% to about 40% by weight of said composition.

10. The composition of 1 wherein said adipic acid is encapsulated.

11. The composition of 10 wherein said adipic acid is encapsulated with maltodextrin or hydrogenated vegetable oil.

12. The composition of 7 wherein said gelatin-frappe has a Ph of about 3.6 or higher.

13. In a process for preparing an ingestible anion exchange resin delivery system composition comprising mixing an anion exchange resin with a confectionery material, the improvement comprising homogeneously incorporating into said composition from abut 0.1% to about 5.0% by weight of adipic acid based on total weight of said composition.

14. The process of 13 comprising incorporating into said composition from about 0.2% to about 1% by weight adipic acid.

15. The process of 13 wherein said anion exchange resin is selected from the group consisting of cholestyramine, colestipol and anion exchange resins having.an imidazolium group.

16. The process of 13 wherein said confectionery material comprises a gelatin-frappe.

17. The process of 16 wherein said gelatin-frappe comprises a mixture of gelatin, water and glycerin.

18. The process of 13 additionally comprising coating said anion exchange resin with lecithin, polyoxyalkylenes, glycerides, polyalkylene glycol, waxes or mixtures thereof.

19. The process of 13 additionally comprising incorporating into said composition, one or more materials selected from the group consisting of sweeteners, flavorings, colorings, humectants, fillers, emulsifiers, thickeners and buffers.

20. The process of 13 comprising incorporating said adipic acid into said composition as an acidulant to adjust the pH of said confectionery material to about 3.6 or higher.

21. The process of 13 wherein said adipic acid is encapsulated.

22. The process of 21 wherein said adipic acid is encapsulated with maltodextrin or hydrogenated vegetable oil.

23. The process of 13 additionally comprising extruding said composition into bar-shaped portions.

## Claims

**1.** An ingestible anion exchange resin delivery system composition comprising anion exchange resin and a confectionery material, characterised in that adipic acid is homogeneously incorporated into the composition in an amount of from about 0.1% to about 5.0% by weight of the total composition.

**2.** The composition of claim 1, wherein adipic acid is present in said composition in an amount of from about 0.2% to about 1% by weight of the composition.

**3.** The composition of claims 1 or 2, wherein said anion exchange resin is selected from the group consisting of cholestyramine, colestipol and anion exchange resins having an imidazolium group.

**4.** The composition of any preceding claim, wherein said confectionery material comprises a gelatin-frappe comprising a mixture of gelatin, water and glycerin.

**5.** The composition of any preceding claim, wherein said anion exchange resin is coated with lecithin, polyoxyalkylenes, glycerides, polyalkylene glycol, waxes or mixtures thereof.

**6.** The composition of any preceding claim, which additionally comprises one or more materials selected from the group consisting of sweeteners, flavourings, colorings, humectants, fillers, emulsifiers, thickeners and buffers.

7. The composition of any preceding claim, wherein said anion exchange resin comprises about 1% to about 40% by weight of said composition.

8. The composition of any preceding claim, wherein the confectionary material has a pH of at least 3.6.

9. The composition of any preceding claim, wherein said adipic acid is encapsulated with a member selected from the group consisting of maltodextrin and hydrogenated vegetable oil.

9. The composition of any preceding claim, when extruded into bar-shaped portions.

10. A process for preparing the composition of any preceding claim, comprising providing an ingestible anion exchange resin delivery system composition comprising anion exchange resin and a confectionery material and optionally one or more materials selected from the group consisting of sweeteners, flavourings, colorings, humectants, fillers, emulsifiers, thickeners and buffers, characterised by homogenously incorporating adipic acid into said composition, in an amount of about 0.1% to about 5.0% by weight of the total composition.

European Patent Office

# EUROPEAN SEARCH REPORT

Application Number

EP 91810154.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US - A - 4 837 255 (F.J. DECHOW) * Claims 1-6; column 2, line 63 - column 3, line 4; example 1 * -- | 1-3, 6-8,11 | A 61 K 31/785<br>A 61 K 9/20<br>A 61 K 9/16<br>A 61 K 47/12 |
| X | EP - A2 - 0 251 369 (PRODOTTI FORMENTI S.R.I.) * Claims 1,8,16; page 3, lines 34 -40; page 4, lines 23-25; page 7, lines 27-40, especially lines 36-40; example 5 * -- | 1,3, 6-8,11 | |
| Y | EP - A2 - 0 190 826 (WARNER-LAMBERT COMPANY) * Claims 1,7,8,12,14; column 7, line 1 - column 9, line 18; example 8 * -- | 1-8,11 | |
| Y | EP - A1 - 0 314 617 (WARNER-LAMBERT COMPANY) * Examples 4-7 in connection with column 7, lines 8-18 * -- | 1-8,11 | TECHNICAL FIELDS SEARCHED (Int. Cl.5) |
| A | US - A - 4 882 157 (R.K. YANG et al.) * Claims 1,6,7,11,12,14; column 8, lines 8-35 * -- | 1,3-8, 10,11 | A 61 K 31/00<br>A 61 K 9/00 |
| A | EP - A1 - 0 261 693 (WARNER-LAMBERT COMPANY) * Claims 1,2,4,5; page 3, lines 5-8,48-49; page 4, lines 24-31,49-58 * -- | 1,3, 5-8,10 | |
| A | US - A - 4 777 042 (H. TODA et al.) * Claims 1,11 * -- | 1,3 | |

The present search report has been drawn up for all claims

| Place of search | Date of completion of the search | Examiner |
|---|---|---|
| VIENNA | 12-06-1991 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

-2-
EP 91810154.4

| Category | Citation of document with indication, where appropriate, of relevant passages | Relevant to claim | CLASSIFICATION OF THE APPLICATION (Int. Cl.5) |
|---|---|---|---|
| A | WO - A1 - 89/12 452 (THE UPJOHN COMPANY) * Claims 1,5; example 1 * ---- | 1,3 | |
| | | | TECHNICAL FIELDS SEARCHED (Int. Cl.5) |

The present search report has been drawn up for all claims

| Place of search | Date of completion of the search | Examiner |
|---|---|---|
| VIENNA | 12-06-1991 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)